(19) Europäisches Patentamt
European Patent Office
Office européen des brevets

(11) Publication number: **0 132 868**
**A1**

# (12) EUROPEAN PATENT APPLICATION

(21) Application number: **84200926.8**

(22) Date of filing: **26.06.84**

(51) Int. Cl.[4]: **C 08 F 2/50**
G 03 F 7/10

(30) Priority: **27.06.83 US 507882**

(43) Date of publication of application:
**13.02.85 Bulletin 85/7**

(84) Designated Contracting States:
**AT BE CH DE FR GB IT LI NL SE**

(71) Applicant: **STAUFFER CHEMICAL COMPANY**

**Westport Connecticut 06880(US)**

(72) Inventor: **Via, Francis Anthony**
**2389 Ridge Street**
**Yorktown Heights New York 10598(US)**

(74) Representative: **Urbanus, Henricus Maria, Ir. et al,**
**c/o Vereenigde Octrooibureaux Nieuwe Parklaan 107**
**NL-2587 BP 's-Gravenhage(NL)**

(54) **Alpha-halogenated aromatic compounds as photoinitiators.**

(57) Alpha-halogenated aromatic compounds of the formula:

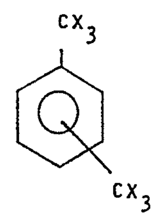

where X is halogen have been found to be photoinitiators for use in the photopolymerization of monomeric and polymeric compositions of photopolymerizable substances. Combination of these compounds with benzoin ether, glyoxylate and/or acetophenone photoinitiator compounds give compositions having enhanced activity at reasonable cost.

EP 0 132 868 A1

Croydon Printing Company Ltd

# ALPHA-HALOGENATED AROMATIC COMPOUNDS AS PHOTOINITIATORS

## Field of the Invention

The present invention relates to processes and compositions for photopolymerization using certain alpha-halogenated aromatic compounds as photoinitiators.

## Background of the Invention

Photopolymerization of unsaturated compositions in which a photoinitiating compound is included in the polymerizable mass is well known in the art. The process has many advantages over thermal polymerization and is particularly useful where long shelf life combined with rapid hardening at low temperature is desirable. Photoinitiating compounds must adsorb light and utilize the energy so acquired to initiate polymerization.

A large number of compounds have been found useful as photoinitiators for the polymerization of unsaturated compounds. Among those heretofore in most common usage in industry are the benzoin ethers of primary and secondary alcohols such as methyl alcohol, ethyl alcohol, isopropyl alcohol and isobutyl alcohol. Also, compounds such as phenyl glyoxal and 1-phenyl butane-1, 2-dione are disclosed as photosensitizers in U. S. Patent No. 2,413,973. Additionally, various acetophenone compounds such as 2,2-diethoxyacetophenone are claimed to have photoinitiating capability in U. S. Patent No. 3,715,293.

In U. S. Patent No. 3,988,152 it was briefly suggested that hexachloro-p-xylene was photosensitive and could be used in conjunction with an organometallic compound. Shahidi et al., in U. S. Patent No. 3,992,275, describe the use of alpha, alpha, alpha-trichlorotoluene as a photoinitiator. More recently, U. S. Patent No. 4,371,606, while indicating at Col. 1, lines 42 - 58 that certain compounds containing halogenomethyl groups can be used as photoinitiators, indicates that they have only low spectral sensitivity and must be excited with the aid of an additional sensitizer.

## Summary of the Present Invention

It has now been found that compounds of the following formula act as photoinitiators:

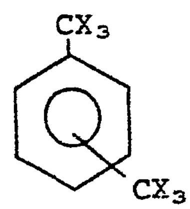

where X is halogen, preferably chlorine.

## Detailed Description of the Present Invention

The compositions curable by actinic radiation according to the invention can contain a photopolymerizable polymer in a reactive ethylenically unsaturated monomeric medium, a reactive polymer alone, a reactive monomer alone, or any of these combined with an inert

solvent. Additionally, the polymerizable composition can contain any of the pigments commonly used in photopolymerization techniques.

The process can be carried out by mixing a quantity of photoinitiating compound of the present invention with a photopolymerizable composition and exposing the resultant mixture to actinic radiation. Alternatively, a one-component system comprising the photopolymerizable composition, the photoinitiator of the invention and, if desired, pigmentation, can be formed.

The photoinitiating compounds of the invention are suitable in the actinic light curing of unsaturated monomeric compounds either alone or as copolymerizable constituents of unsaturated polymer/monomer systems. Such systems are composed of mixtures of conventional unsaturated polymers and unsaturated monomers.

Monomers which are useful in practicing the invention are acrylic, α-alkacrylic and α-chloroacrylic acid compounds such as esters, amides and nitriles. Examples of such compounds are acrylonitrile, methacrylonitrile, methyl acrylate, ethyl acrylate, methyl methacrylate, isobutyl methacrylate, 2-ethylhexyl acrylate, methacrylamide and methyl α-chloroacrylate. Also useful, although not preferred due to their slower rates of reactivity, are vinyl and vinylidene esters, ethers and ketones. Additionally, compounds having more than one terminal unsaturation can be used. Examples of these include diallyl phthalate, diallyl maleate, diallyl fumarate, triallyl cyanurate, triallyl phosphate, ethylene glycol dimethacrylate, glycerol trimethacrylate, pentaerythritol triacrylate,

pentaerythritol tetraacrylate, trimethylolpropane triacrylate, trimethylolpropane triacrylate, methacrylic anhydride, and allyl ethers of monohydroxy or polyhydroxy compounds, such as ethylene glycol diallyl ether, pentaerythritol tetraallyl ether, and the like. Non-terminally unsaturated compounds such as diethyl fumarate can similarly be used.

The acrylic acid derivatives are particularly well suited to the practice of the invention and are consequently preferred components as monomers in monomer-containing polymerizable systems and as reactive centers in polymerizable polymers. While monomeric styrene can be used in the practice of the invention, it is not a preferred constituent of systems polymerizable thereby due to its slow rate of reaction.

A preferred manner of practicing the invention is by the use of photopolymerizable molding and coating compositions which comprise mixtures of unsaturated polymeric compounds and monomeric compounds copolymerizable therewith. The polymeric compounds can be conventional polyesters prepared from unsaturated polycarboxylic acids such as maleic acid, fumaric acid, glutaconic acid, itaconic acid, citraconic acid, mesaconic acid and the like, and polyhydric alcohols such as ethylene glycol, diethylene glycol, glycerol, propylene glycol, 1,2-butanediol, 1,4-butanediol, pentaerythritol, trimethylolpropane and the like. The carboxylic acid content can also contain saturated components. The inclusion of a monobasic fatty acid content, either as such or in the form of a triglyceride or oil, in the photopolymerizable polyester composition to comprise an alkyd resin is also acceptable. These resins can, in turn, be modified

by silicones, epoxides, isocyanates, etc., by known techniques.

The alpha halogenated aromatic photoinitiators of the present invention, which are rather economical compounds to produce, can be combined with at least one more expensive known glyoxylate, benzoin ether or acetophenone type photoinitiator, for example, to yield photoinitiator combinations of enhanced activity at reasonable cost.

Glyoxylate photoinitiators are known compounds and possess the glyoxylate group ( -C(O)-C(O)-O- ) attached to suitable moieties which confer photoinitiator utility on the resulting compound. Compounds of this type are described in U.S. Patent 4,038,164 to F. A. Via, which is incorporated herein by reference. That patent describes compounds of the formula

$$R'-\overset{\overset{\displaystyle O}{\|}}{C}-\overset{\overset{\displaystyle O}{\|}}{C}-OR$$

where R can be a straight or branched chain hydrocarbon (e.g., $C_1$-$C_{10}$ alkyl) of from one to ten carbon atoms, aryl, aralkyl, or mono- di, or trisilyl and R' is a heterocyclic radical, aryl of from 6 to 14 carbon atoms, or mono-, di- or polysubstituted phenyl with such substituents as alkyl, alkoxy, aryloxy, alkylthio, arylthio and halogen. Preferably R is alkyl and R' is phenyl. A commercial product of this type is sold under the trademark VICURE-55 by Stauffer Chemical Company and contains methyl phenyl glyoxylate as the photoinitiator.

The terminology "benzoin ether" as used herein is intended to cover those compounds that have photoinitiator action which contain at least one -OR group

on the alpha carbon atom adjacent the carbonyl group. Such compounds are of the formula

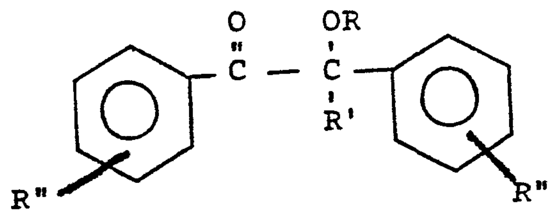

where R is alkyl, R' is either hydrogen, alkyl, halogen or alkoxy, and where R" is independently alkyl, alkoxy, halogen and alkylamino. More than one R" group can be present on either of the phenyl rings to substitute for the hydrogen atom normally present. When R' is alkoxy, these compounds can also be called the ketals of benzil.

Commercial photoinitiators of this type are sold under the trademarks VICURE-10 (R = isobutyl and R' = hydrogen) and VICURE-30 (R = isopropyl and R' = hydrogen) by Stauffer Chemical Company and IRGACURE 651 (R = methyl and R' = methoxy) by Ciba-Geigy Corporation.

Also includable are acetophenone photoinitiators of the formula

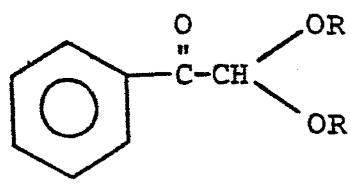

where R is lower alkyl (e.g., $C_1 - C_6$ alkyl). An example is diethoxyacetophenone.

In such mixtures, the amount of alpha-chlorinated aromatic compound of the instant invention is present at about 25% by weight, or greater, of the mixture of that compound and the selected benzoin ether, glyoxylate, acetophenone photoinitiator or

photoinitiators. A preferred amount is a weight ratio of from about 40:60 to about 99:1 of alpha-chlorinated compound to known photoinitiating compound or compounds.

Additionally, the photopolymerizable composition can contain a sensitizer capable of enhancing the photoinitiating reactivity of the photoinitiating compound of the invention. Examples of sensitizers useful in the practice of the invention are such compounds as xanthone, thioxanthone, acetophenone and the like. These are typically added in amounts ranging from about 0.1 to about 6 weight percent. The techniques whereby such sensitizers are selected for use in conjunction with particular photoinitiators are well known in the art. See for example, Murov, Handbook of Photochemistry, Marcel Dekker, Inc., New York (1973).

Thus it is seen that the constitution of photopolymerizable compositions which can be used in the practice of the invention is widely variable. However, the compounds enumerated above are purely illustrative. Materials subject to polymerization by actinic radiation as well as permissible variations and substitutions of equivalent components within particular types of compositions are well known to those skilled in the art.

The compounds which function as photoinitiators in accordance with the present invention are known for other uses and are easily synthesized by known means.

The photoinitiators of the invention can be utilized in amounts ranging from 0.01 to about 30% by weight based on the photopolymerizable composition. However, preferable amounts of the compounds are from about 0.5 to about 20 weight percent with optimal

results being achieved with amounts in the range of from about 1.0 to about 16 weight percent.

An acceptable source of actinic light radiation is any apparatus which emits light radiation in the approximate region of from about 2000 Angstroms to about 8000 Angstroms and preferably from about 2400 Angstroms to about 5400 Angstroms. One such apparatus is PPG Model QC 1202, a UV processor, manufactured by PPG Industries, Inc.

The present invention is illustrated by the Examples which follow.

TEST PROCEDURE USED IN THE EXAMPLES

The induced rate of photopolymerization of a standard test solution of acrylate/alkyd resin was measured for each of the compounds tested. This standard test solution consisted of 42%, by weight, trimethylolpropane triacrylate (TMPTA), 17%, by weight, of ethylhexyl acrylate (EHA), and 41%, by weight, of an unsaturated long chain linseed oil alkyd resin (ACTOMER X-80 brand from Union Carbide).

The test solution was exposed to light radiation from a light source (PPG Model QC 1202) which contained two high intensity, medium pressure quartz mercury lamps, 12 inches in length, with each operating at a linear power density of about 200 watts per inch or 2400 watts per lamp. The lamps were housed in an elliptical reflector above a variable speed conveyor belt so that each lamp provided a 2-inch band of high flux actinic radiation on the conveyor. This 2-inch exposure area was bordered on both sides by an additional 2-inch area of medium flux energy for a total radiation area of 6 inches for each lamp. In the curing data presented herein, the cure rate of the polymerizable compositions is presented in feet-per-minute-per-lamp (ft/min/lamp). Thus, a conveyor belt speed of one foot/min. will, with a 12-inch exposure area for the two lamps, provide 60 seconds of exposure or a cure rate of 0.5 ft./min./lamp. Similarly, a belt speed of 10 ft./min. will provide 6 seconds of exposure or a rate of 5 ft./min./lamp, while a speed of 20.0 ft.min. will give 3 seconds exposure or a rate of 10 ft./min./lamp.

The extent of curing was determined by a standard pencil hardness test with all samples being coated on aluminum plate to a thickness of 2 mils and polymerized to achieve a standard pencil hardness of from 4H to 6H where this was attainable.

EXAMPLE 1

This Example illustrates the photoefficiency of various alpha-chlorinated aromatic compounds when used at 4 wt. % loading in the standard test solution:

| Compound | Cure Rate (feet/min./lamp) |
| --- | --- |
| alpha, alpha, alpha, alpha', alpha', alpha'-hexachloro-*m*-xylene | 8 |
| alpha, alpha, alpha, alpha' alpha', alpha'-hexachloro-*p*-xylene | 9 |

EXAMPLE 2

This Example illustrates the effects of concentration on the reactivity of the meta-xylene initiator compound of Example 1 in accordance with the present invention.

| Initiator Concentration (wt. %) | Cure Rate (feet/min./lamp) |
|---|---|
| 4 | 9 |
| 8 | 20 |
| 12 | 10 |
| 16 | 5 |
| 20 | 2 |

EXAMPLE 3

This Example illustrates the photoefficiency of certain known photoinitiators and an alpha-chlorinated aromatic as described herein. It also illustrates the superior effect found when the alpha-chlorinated compound is combined with those known photoinitiators. All were at 4 wt. % loading in the test solution:

| Compound | Cure Rate (feet/min. |
|---|---|
| VICURE-10 photoinitiator* | 8 |
| DEAP*** | 8 |
| VICURE-55 photoinitiator** | 15 |
| Compound of Example 2 | 8 |
| VICURE-10 + Compound of Example 2 | 20 |
| DEAP + Compound of Example 2 | 20 |
| VICURE-55 + Compound of Example 2 | 30 |

* VICURE-10 brand is available from Stauffer Chemical Company and contains, as the active ingredient, benzoin i-butyl ether.

** VICURE-55 brand is also available from Stauffer Chemical Company and contains, as the active ingredient, methyl phenyl glyoxylate.

*** DEAP is an abbreviation for diethoxyacetophenone.

EXAMPLE 4

This Example illustrates the effect of an alpha-chlorinated compound as a photoinitiator alone and mixed with certain promoter compounds. The alpha-chlorinated compound was the compound tested in Example 2. It was present at 4 wt. % of the test solution and the promoter at 2 wt. %.

| Promoter | Cure Rate (feet/min./lamp) |
|---|---|
| None | 9 |
| $C_6H_5CH_2OH$ | 11 |
| $C_6H_5CH_2S(O)(CH_2CH_6H_5)$ | 15 |
| ERL-4221* | 15 |
| Mercaptate ester Q-43** | 20 |
| $C_6H_5CH_2OCH_2C_6H_5$ | 15 |

* 3,4-epoxy cyclohexylmethyl-3,4-epoxy-cyclohexane carboxylate, available from Union Carbide Corp.

** pentaerythritol tetrakis (mercapto propionate), available from Cincinnati Milacron Chemicals.

EXAMPLE 5

This Example shows the performance range of the compound of Example 2 as a photoinitiator in various systems described below. The concentration of photoinitiator was at 4 wt. %.

| System | Cure Rate (feet/min./lamp) |
|---|---|
| A | 5 |
| B | 12 |
| C | 25 |
| D | 16 |
| E | 25 |

System A: 50 wt. % trimethylolpropane triacrylate and 50 wt. % UVIMER-540 resin (49 parts urethane oligomer, 19 parts hydroxyethyl acrylate and 32 parts pentaerythritol tetraacrylate), available from Polychrome.

System B: 50 wt. % trimethylolpropane triacrylate and 50 wt. % EPOCRYL Resin DRH-303 available from Shell Chemical Company. The EPOCRYL brand resin is a diacrylate ester of bisphenol A.

System C: 50 wt. % of trimethylolpropane triacrylate and 50 wt. % of acrylated linseed oil (ACTOMER X-80 brand from Union Carbide Corp.).

System D: 50 wt. % of 1,6-hexanediol diacrylate and 50 wt. % of ACTOMER X-80 resin.

System E: 100 wt. % of pentaerythritol triacrylate.

## EXAMPLE 6

This Example illustrates various promoters used with two of the alpha-chlorinated initiators of the present invention. The initiator was at 4 wt. %; the promoter at 2 wt. %:

| Promoter | Cure Rate A | Cure Rate B |
|---|---|---|
| None | 8 | 8 |
| VICURE-10 | 15 | 15 |
| Vanadyl naphthanate | 16 | 15 |
| Mercaptate ester Q-43 | 20 | 15 |
| ERL-4221 | 11 | 10 |
| Cyclohexene oxide | 10 | -- |
| 1,2-epoxydecane | 11 | -- |
| Styrene oxide | 10 | -- |
| Benzyl sulfoxide | 15 | -- |

A: the meta-xylene compound of Example 1 was used as initiator.

B: the para-xylene compound of Example 1 was used as initiator.

The foregoing Examples are intended to merely be illustrative of certain embodiments of the present invention and should not be construed in a limiting manner. The scope of protection that is sought is set forth in the claims which follow.

What is Claimed:

1. In the photopolymerization of monomeric and polymeric compositions of photopolymerizable substances wherein a photoinitiator is admixed with a photopolymerizable composition and the mixture exposed to actinic radiation, the improvement wherein photopolymerization is effectively initiated by a compound of the formula:

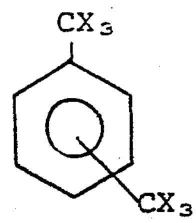

with X being halogen.

2. A method as claimed in Claim 1 where X is chlorine.

3. A method as claimed in Claim 1 wherein the compound is alpha, alpha, alpha, alpha', alpha', alpha'-hexachloro-m-xylene.

4. A method as claimed in Claim 1 wherein the compound is alpha, alpha, alpha, alpha', alpha', alpha'-hexachloro-p-xylene.

5. The method of Claim 2 wherein the compound is present at a concentration of about 0.5 percent by weight to about 20 percent by weight.

6. The method of Claim 2 wherein the compound is present at a concentration of about 1.0 percent by weight to about 16 percent by weight.

7. The method of Claim 3 wherein the compound is present at a concentration of about 0.5 percent by weight to about 20 percent by weight.

8. The method of Claim 3 wherein the compound is present at a concentration of about 1.0 percent by weight to about 16 percent by weight.

9. The method of Claim 4 wherein the compound is present at a concentration of about 0.5 percent by weight to about 20 percent by weight.

10. The method of Claim 4 wherein the compound is present at a concentration of about 1.0 percent by weight to about 16 percent by weight.

11. The method of Claim 1 wherein the compound is combined with a photoinitiator compound selected from the group consisting of benzoin ether, glyoxylate, and acetophenone compounds such that the compound is present in at least 25%, by weight of the composition.

12. The method of Claim 11 wherein the compound is a benzoin ether of the formula:

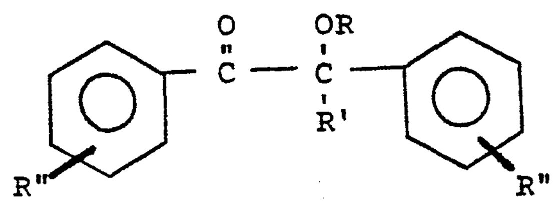

where R is alkyl and R' is selected from the group consisting of hydrogen, alkyl, halogen, and alkoxy and R" is selected from the group consisting of alkyl, alkoxy, halogen and alkylamino.

13. The method of Claim 11 wherein the benzoin ether is benzoin isobutyl ether.

14. The method of Claim 11 wherein the benzoin ether is benzoin isopropyl ether.

15. The method of Claim 11 wherein the compound is a glyoxylate of the formula:

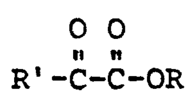

where R is $C_1$-$C_{10}$ alkyl and R' is phenyl.

C-4860-IV

16. The method of Claim 11 wherein the compound is methyl phenyl glyoxylate.

17. The method of Claim 11 wherein the compound is an acetophenone of the formula:

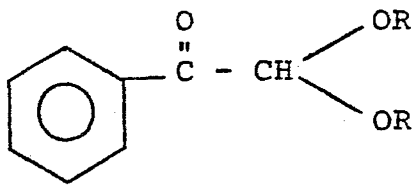

where R is lower alkyl.

18. The method of Claim 11 wherein the compound is diethoxyacetophenone.

19. A composition photopolymerizable by actinic radiation comprising unsaturated polymerizable constituents containing dispersed therein an effective amount of a photoinitiating compound of the formula:

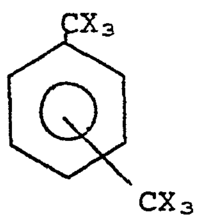

where X is halogen.

20. A composition as claimed in Claim 19 where X is chlorine.

21. A composition as claimed in Claim 19 where the compound is alpha, alpha, alpha, alpha', alpha', alpha'-hexachloro-m-xylene.

22. A composition as claimed in Claim 19 where the compound is alpha, alpha, alpha, alpha', alpha', alpha'-hexachloro-p-xylene.

23. The composition of Claim 20 wherein the compound is present at a concentration of about 0.5 percent by weight to about 20 percent by weight.

24. The composition of Claim 20 wherein the compound is present at a concentration of about 1.0 percent by weight to about 16 percent by weight.

25. The composition of Claim 21 wherein the compound is present at a concentration of about 0.5 percent by weight to about 20 percent by weight.

26. The composition of Claim 21 wherein the compound is present at a concentration of about 1.0 percent by weight to about 16 percent by weight.

27. The composition of Claim 22 wherein the compound is present at a concentration of about 0.5 percent by weight to about 20 percent by weight.

28. The composition of Claim 22 wherein the compound is present at a concentration of about 1.0 percent by weight to about 16 percent by weight.

29. The composition of Claim 19 wherein the compound is combined with a photoinitiator compound selected from the group consisting of benzoin ether, glyoxylate, and acetophenone compounds such that the compound is present in at least 25%, by weight of the composition.

30. The composition of Claim 29 wherein the compound is a benzoin ether of the formula:

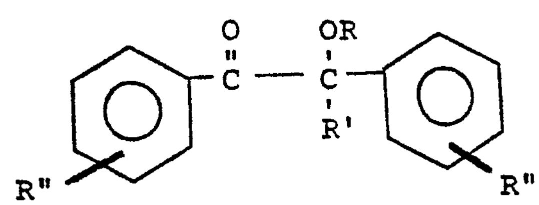

where R is alkyl and R' is selected from the group consisting of hydrogen, alkyl, halogen, and alkoxy and R" is selected from the group consisting of alkyl, alkoxy, halogen, and alkylamino.

31. The method of Claim 29 wherein the benzoin ether is benzoin isobutyl ether.

32. The method of Claim 29 wherein the benzoin ether is benzoin isopropyl ether.

33. The method of Claim 29 wherein the compound is a glyoxylate of the formula:

$$R'-\overset{\overset{O}{\|}}{C}-\overset{\overset{O}{\|}}{C}-OR$$

where R is $C_1$-$C_{10}$ alkyl and R' is phenyl.

34. The method of Claim 29 wherein the compound is methyl phenyl glyoxylate.

35. The method of Claim 29 wherein the compound is an acetophenone of the formula:

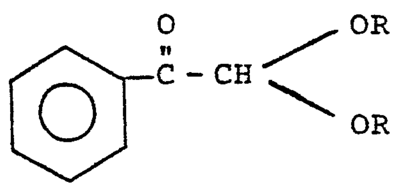

where R is lower alkyl.

36. The method of Claim 29 wherein the compound is diethoxyacetophenone.

European Patent Office

# EUROPEAN SEARCH REPORT

0132868
Application number

EP 84 20 0926

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-3 251 759 (H.R. ANDERSON)<br>* Claims 1,11; example 1 *<br>----- | 1-36 | C 08 F 2/50<br>G 03 F 7/10 |
| | | | TECHNICAL FIELDS SEARCHED (Int. Cl. 3) |
| | | | C 08 F<br>G 03 F |

The present search report has been drawn up for all claims

| Place of search | Date of completion of the search | Examiner |
|---|---|---|
| THE HAGUE | 17-10-1984 | CAUWENBERG C.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82